(19) **Europäisches Patentamt / European Patent Office / Office européen des brevets**

(11) **EP 1 385 470 B1**

(12) **EUROPÄISCHE PATENTSCHRIFT**

(45) Veröffentlichungstag und Bekanntmachung des Hinweises auf die Patenterteilung:
**08.08.2007   Patentblatt 2007/32**

(51) Int Cl.:
***A61Q 13/00*** (2006.01)

(21) Anmeldenummer: 02703629.2

(22) Anmeldetag: **11.03.2002**

(86) Internationale Anmeldenummer:
**PCT/EP2002/002647**

(87) Internationale Veröffentlichungsnummer:
**WO 2002/076421 (03.10.2002 Gazette 2002/40)**

(54) **RIECHSTOFFMISCHUNGEN MIT GESTEIGERTER GERUCHSTÄRKE**

FRAGRANT MIXTURES WITH INCREASED STRENGTH OF ODOR

MELANGES DE PARFUMS A ODEUR PLUS INTENSE

(84) Benannte Vertragsstaaten:
**AT BE CH CY DE DK ES FI FR GB GR IE IT LI LU MC NL PT SE TR**

(30) Priorität: **23.03.2001   JP 2001086046**

(43) Veröffentlichungstag der Anmeldung:
**04.02.2004   Patentblatt 2004/06**

(73) Patentinhaber: **Symrise GmbH & Co. KG**
**37603 Holzminden (DE)**

(72) Erfinder:
• **KAWAKAMI, Ken**
**Yokohama-shi, Kanagawa 226-0027 (JP)**
• **SONNENBERG, Steffen**
**37603 Holzminden (DE)**
• **MCDERMOTT, Keith**
**Bound Brook, NJ 08805 (US)**
• **SMITH, Leslie**
**Princeton, NJ 08540 (US)**

(74) Vertreter: **Eisenführ, Speiser & Partner**
**Patentanwälte Rechtsanwälte**
**Postfach 10 60 78**
**28060 Bremen (DE)**

(56) Entgegenhaltungen:
**EP-A- 0 908 174          WO-A-00/01360**
**US-A- 6 024 943          US-A- 6 086 903**

• **PATENT ABSTRACTS OF JAPAN vol. 2000, no. 08, 6. Oktober 2000 (2000-10-06) & JP 2000 135111 A (TAKANA KOICHIRO), 16. Mai 2000 (2000-05-16)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die vorliegende Erfindung betrifft die Verwendung von Riechstoffmischungen in Haarpflegemitteln sowie Haarpflegemittel enthaltend diese Riechstoffmischungen. Der Geruch der erfindungsgemäßen Riechstoffmischungen wird beim bzw. nach dem Trocknen der Haare besonders stark wahrgenommen.

Die Verwendung eines Haartrockners zum Legen oder Trocknen von Haar ist üblich. Der bei der Verwendung eines Haartrockners vom Haar vorteilhaft mittels eines warmen Luftstroms freigesetzte Duft bewirkt Frische, ein angenehmes Gefühl sowie Attraktivität. Herkömmlicherweise werden Riechstoffmischungen für Haarpflegemittel mit dem Ziel eingesetzt, Produkten einen bevorzugten Geruch zu verleihen, beim Vorgang des Waschens von Haar Duft freizusetzen oder einen Duft auf dem trockenen Haar freizusetzen.

Gewöhnlich weisen die meisten in Haarpflegemitteln wie einem Shampoo, einer Haarspülung, einem Haarconditioner oder einer Haarkur enthaltenen Riechstoffe die Neigung auf, während des Vorgangs des Haarewaschens verloren (z.B. durch Ausspülen) zu gehen. Darüber hinaus weisen einige der auf dem feuchten und trockenen Haar verbleibenden Riechstoffe, wenn sie durch einen warmen Luftstrom erwärmt werden, keine ausreichende Flüchtigkeit auf. Folglich ergeben die herkömmlichen Riechstoffmischungen bei der Trocknung des Haares bei erhöhter Temperatur, z.B. mit einem Haartrockner, nicht die gewünschte geruchliche Wirkung.

Es besteht daher der Bedarf an Haarpflegemitteln mit Riechstoffmischungen, die nach der Anwendung von Haarpflegemitteln einen deutlich gesteigerten Duft beim Trocknen des Haares bei erhöhter Temperatur freisetzen.

Es wurde nun überraschenderweise gefunden, dass bestimmte Riechstoffmischungen einen deutlich intensiveren, stärkeren Geruch, d.h. eine gesteigerte Freisetzung von Riechstoffen, bei und nach der Trocknung von nassem Haar bei erhöhter Temperatur ermöglichen.

Gegenstand der vorliegenden Erfindung sind Haarpflegemittel gemäß Anspruch 1 und dessen Unteransprüchen. Die Haarpflegemittel enthalten Riechstoffmischungen mit mindestens 5 Riechstoffen mit einem Freisetzungsindex von größer oder gleich 4 und einer Geruchsstärke von größer oder gleich 2,5, mit der Maßgabe, dass der Gesamtanteil dieser Riechstoffe an der Gesamt-Riechstoffmischung 20 bis 100 Gew.% beträgt.

Unter dem Freisetzungsindex wird in der vorliegenden Erfindung der Quotient der gaschromatographisch ermittelten Peakfläche (GC-Peakfläche) eines Riechstoffes in einer Gasraum-Probe über dem frisch getrockneten Haar bei 50°C und bei 25°C verstanden und nach folgender Formel berechnet:

$$\text{Freisetzungsindex} = [\text{GC-Peakfläche bei 50°C}] / [\text{GC-Peakfläche bei 25°C}]$$

Der Gehalt des Riechstoffes korreliert mit der GC-Peakfläche. Riechstoffe mit einem hohen Freisetzungsindex werden beim Erhitzen auf 50°C besonders stark vom Haar freigesetzt und ergeben somit einen starken Geruchseindruck.

Unter der Geruchsstärke wird in der vorliegenden Erfindung die geruchliche Intensität des vom gewaschenen Haar oder Haarmuster freigesetzten Riechstoffes bzw. der freigesetzten Riechstoffmischung verstanden. Die Bewertung der Geruchsstärke erfolgte von einer Sachverständigengruppe anhand der folgenden Skala: 5 - sehr stark, 4 - stark, 3 - mittel, 2 - schwach, 1 - kein Geruch.

Bevorzugt enthalten die erfindungsgemäßen Riechstoffmischungen mindestens 6, besonders bevorzugt mindestens 7 Riechstoffe mit einem Freisetzungsindex von größer oder gleich 4 und einer Geruchsstärke von größer oder gleich 2,5.

Bevorzugt sind Riechstoffe mit einem Freisetzungsindex von größer oder gleich 5, besonders bevorzugt solche mit einem Freisetzungsindex von größer oder gleich 6 und ganz besonders bevorzugt solche mit einem Freisetzungsindex von größer oder gleich 7.

Bevorzugt sind Riechstoffe mit einer Geruchsstärke von größer oder gleich 3, besonders bevorzugt solche mit einer Geruchsstärke von größer oder gleich 3,4 und ganz besonders bevorzugt solche mit einer Geruchsstärke von größer oder gleich 3,8.

Eine gesteigerte Freisetzung dieser Riechstoffe und dadurch eine stärkere, intensivere Geruchswahrnehmung während und nach der Trocknung der Haare, findet besonders bei Temperaturen oberhalb von 40°C statt, ganz besonders bei Temperaturen oberhalb von 50°C.

Die in den erfindungsgemäßen Haarpflegemitteln enthaltenen Riechstoffe können in die drei verschiedenen Geruchsgruppen A, B und C unterteilt werden. Die Gruppe A beinhaltet Riechstoffe mit frischem, grünem, zitrusartigem und herbem Geruch. Diese Riechstoffe sind sehr häufig in der Kopfnote einer Riechstoffmischung enthalten. Die Gruppe B beinhaltet Riechstoffe mit allen blumigen Geruchsrichtungen. Diese Riechstoffe sind sehr häufig in der Herznote einer Riechstoffmischung enthalten. Die Gruppe C beinhaltet alle Riechstoffe mit holzigem, ambriertern und moschusartigem Geruch. Diese Riechstoffe sind sehr häufig im Fond oder der Basisnote einer Riechstoffmischung enthalten.

Riechstoffgemische für Haarpflegemittel im Sinne der Erfindung enthalten vorteilhafterweise mindestens zwei Riechstoffe der Gruppe B und mindestens einen der Geruchsgruppe A oder der Geruchsgruppe C.

Bevorzugt enthalten die Riechstoffgemische mindestens 3, besonders bevorzugt mindestens 4 Riechstoffe der Gruppe B.

Wenn die Riechstoffgemische nur Riechstoffe der Gruppe A und der Gruppe B oder nur der Gruppe B und der Gruppe C enthalten; so sind Riechstoffgemische bevorzugt, die mindestens jeweils zwei Riechstoffe aus den genannten Gruppen enthalten.

Vorteilhaft sind erfindungsgemäß Riechstoffgemische, die mindestens zwei Riechstoffe der Gruppe B und mindestens je einen Riechstoff der Gruppen A und C enthalten.

Zur Erzielung eines besonders starken Geruchseindruckes ist es vorteilhaft, bestimmte Gewichtsverhältnisse der einzelnen Geruchsgruppen A, B und C zu wählen. Die Gewichtsverhältnisse beziehen sich auf die Summe der Riechstoffmengen der j'e-weiligen Geruchsgruppe. Im Falle, dass alle drei. Geruchsgruppen A, B und C in der erfindungsgemäß eingeselzten Riechstoffmischung verwendet werden, liegt das Gewichtsverhältnis von A : B bevorzugt im Bereich von 1 : 2 bis 1 : 4, und das Verhältnis von B : C bevorzugt im Bereich von 1 : 1 bis 4 : 1.

In Falle, dass nur Riechstoffe der Geruchsgruppen A und B verwendet werden, liegt das Verhältnis von A : B bevorzugt im Bereich von 1 : 1 bis 1 : 4.

In Falle, dass nur Riechstoffe der Geruchsgruppen B und C verwendet werden, liegt das Verhältnis von B : C bevorzugt im Bereich von 1 : 2 bis 5 : 1.

Bevorzugte Riechstoffe, die in den erfindungsgemäßen Riechstoffmischungen enthalten sein können, sind in Tabelle 1 aufgeführt.

**Tabelle 1:**

| Gruppe | Bevorzugte Riechstoffe |
|---|---|
| A | Acetaldehyd-2-phenyl-2,4-pentandiolacetal |
| A | 2,6,10-Trimethyl-9-undecenal |
| A | 2-Propenylcyclohexyloxyacetat |
| A | Dodecanal |
| A | 1-(3-Methyl-2-benzofuranyl)ethanon |
| B | Gamma-Undecalacton |
| B | 3-(4-tert.-Butylphenyl)propanal |
| B | 4-(4-Methyl-3-penten-1-yl)-3-cyclohexen-1-carboxaldehyd |
| B | 2-Methyl-3-(4-tert.-butylphenyl)propanal |
| B | 2-Methyl-3-(4-isopropylphenyl)propanal |
| B | 3-(3-Isopropylphenyl)butanal |
| B | Beta-Damascenon |
| B | Beta-Damascon |
| B | Delta-Damascon |
| B | 5-Hexyldihydro-5-methyl-2(3H)-furanon |
| B | Alpha-Hexylzimtaldehyd |
| B | Alpha-Iron |
| B | Beta-Ionon |
| B | Gamma-Methylionon |
| B | Methyl-2-pentyl-3-oxocyclopentylacetat |
| B | 1-(2-Benzofuranyl)ethanon |
| B | Heliotropin |
| C | 3-Methylcyclopentadecenon |

(fortgesetzt)

| Gruppe | Bevorzugte Riechstoffe |
|--------|------------------------|
| C | 1-(1,2,3,4,5,6,7,8-Octahydro-2,3,8,8-tetramethyl-2-naphthyl)ethanon |
| C | 1-(1,2,3,4,5,6,7,8-Octahydro-2,3,5,5-tetramethyl-2-naphthyl)ethanon |
| C | Acetylcedren |
| C | Oxacyclohexadecen-2-on |
| C | Dodecahydro-3a,6,6,9a-tetramethylnaphtho[2,1-b]furan |
| C | Cyclopentadecanolid |

Besonders bevorzugte Riechstoffe, die in den erfindungsgemäßen Riechstoffmischungen enthalten sein können, sind aus Gruppe A: 2,6,10-Trimethyl-9-undecenal, 2-Propenylcyclohexyloxyacetat und 1-(3-Methyl-2-benzofuranyl) ethanon; aus Gruppe B: Gamma-Undecalacton, 2-Methyl-3-(4-tert.-butylphenyl)propanal, 3-(3-Isopropylphenyl)butanal, 5-Hexyldihydro-5-methyl-2(3H)-furanon, Alpha-Hexylzimtaldehyd, Alpha-Iron, Beta-Ionon, Gamma-Methylionon, Methyl-2-pentyl-3-oxocyclopentylacetat, 1-(2-Benzofuranyl)ethanon und Heliotropin; aus Gruppe C: 3-Methylcyclopentadecenon, 1-(1,2,3,4,5,6,7,8-Octahydro-2,3,8,8-tetramethyl-2-naphthyl)ethanon, 1-(1,2,3,4,5,6,7,8-Octahydro-2,3,5,5-tetramethyl-2-naphthyl)ethanon, Acetylcedren, Oxacyclohexadecen-2-on, Dodecahydro-3a,6,6,9a-tetramethylnaphtho [2,1-b]furan und Cyclopentadecanolid.

Der Anteil der Riechstoffmischung bezogen auf die in dem Haarpflegemittel enthaltene Gesamt-Riechstoffmischung liegt zwischen 20 und 100 Gew.%. Unterhalb eines Anteils von 10 Gew.% an der Gesamt-Riechstoffmischung war kein wesentlicher Beitrag der Riechstoffmischung im Sinne der Erfindung feststellbar. Bevorzugt ist ein Anteil der Riechstoffmischung in der Gesamt-Riechstoffmischung von mindestens 25 Gew.%, besonders bevorzugt von mindestens 30 Gew.%, und ganz besonders bevorzugt mindestens 40 Gew.%. In einer weiteren besonders bevorzugten Ausführungsform beträgt der Anteil der erfindungsgemäß zusammengesetzten Riechstoffmischung an der Gesamt-Riechstoffmischung mindestens 50.Gew.% und in einer ganz besonders bevorzugten Ausführungsform mindestens 60 Gew.%.

Die in dem Haarpflegemittel enthaltene Gesamt-Riechstoffmischung kann neben den erfindungsgemäßzusammengesetzten Riechstoffmischungen weitere Riechstoffe enthalten. Beispiele für Riechstoffe, mit denen die erfindungsgemäßen Riechstoffimischungen vorteilhaft kombiniert werden können, finden sich z.B. in K. Bauer, D. Garbe und H. Surburg, Common Fragrance and Flavor Materials, 3rd. Ed., Wiley-VCH, Weinheim 1997.

Im Einzelnen seien beispielhaft genannt:

Extrakte aus natürlichen Rohstoffen wie Etherische Öle, Concretes, Absolues, Resine, Resinoide, Balsame, Tinkturen, sowie Fraktionen davon bzw. daraus isolierte Inhaltsstoffe; Einzel-Riechstoffe aus der Gruppe der Kohlenwasserstoffe; der aliphatischen Alkohole; der aliphatischen Aldehyde und deren 1,4-Dioxacycloalken-2-one; der aliphatischen Ketone und deren Oxime; der aliphatischen schwefelhaltigen Verbindungen; der aliphatischen Nitrile; der aliphatischen Carbonsäuren und deren Ester; der acyclischen oder cyclischen Terpenalkohole sowie deren Formiate, Acetate, Propionate, Isobutyrate, Butyrate, Isovalerianate, Pentanoate, Hexanoate, Crotonate, Tiglinate, 3-Methyl-2-butenoate; der acyclischen oder cyclischen Terpenaldehyde und -ketone; der cyclischen, araliphatischen oder cycloaliphatischen Alkohole; der cyclischen und cycloaliphatischen Ether; der cyclischen und makrocyclischen Ketone; der cycloaliphatischen Aldehyde und Ketone; der Ester cyclischer oder cycloaliphatischer Alkohole; der aromatischen Kohlenwasserstoffe; der Ester von araliphatischen Alkoholen und aliphatischen Carbonsäuren; der araliphatischen Ether; der aromatischen und araliphatischen Aldehyde; der aromatischen und araliphatischen Ketone; der aromatischen und araliphatischen Carbonsäuren und deren Ester; der stickstoffhaltigen aromatischen Verbindungen, der Phenole, Phenylether und Phenylester; der heterocyclischen Verbindungen, der Lactone und der makrocyclischen Lactone.

Als Haarpflegemittel im Sinne der Erfindung seien beispielsweise genannt Shampoo, Haarspülung, Haarconditioner, Haarkur, Haarspray, Haarlotion, Haarpflegeschaum, Haaröl, Haarwasser, Haarcreme, Haargel, Pomade, permanentes oder semipermanentes Haarfärbemittel, Haartonikum oder Haarwuchsmittel. Bevorzugte Haarpflegemittel sind Shampoo, Haarspülung, Haarconditioner und Haarkur.

Weitere Inhaltstoffe, die in der Formulierung des Haarpflegemittels enthalten sein können sind beispielsweise Tenside, Silicone, kationische Polymere, Feuchthaltemittel, Erweichungsmittel, Adstringentien, Haarwuchsmittel, juckreizlindernde Mittel, entzündungshemmende Mittel, Alkohole, Polyole, Verdickungsmittel, farbgebende Mittel, Sonnenschutzfilter, Chelatbildner, Antioxidantien, antimikrobielle Mittel, Konservierungsmittel, Erdöldestillate und Pflanzenextrakte und sonstige übliche kosmetische Zusätze.

4

Die erfindungsgemäßzusammengesetzten Riechstoffmischungen können mit kosmetischen Zusätzen zu einem Haarpflegemittel vermischt werden. Der Anteil der erfindungsgemäßen Riechstoffmischung bezogen auf das Gesamtgewicht des Haarpflegemittels liegt typischerweise im Bereich von 0,01 bis 2,0 Gew.%, bevorzugt im Bereich von 0,1 bis 1,5 Gew.%, und besonders bevorzugt im Bereich von 0,2 bis 1, 0 Gew.%.

Die erfindungsgemäßen Haarpflegemittel können beispielsweise in Form von Flüssigkeiten, Dispersionen, Milch, Sprays, Gels, Stiften oder Emulsionen vom Öl-in-Wasser-, vom Wasser-in-Öl-, vom Öl-in-Wasser-in-Öl- und vom Wasser-in-Öl-in-Wasser-Typ wie z.B. Cremes oder Lotionen vorliegen.

Die Erfindung kann anhand folgender Beispiele erläutert werden.

## Beispiel 1

Ein unparfümiertes Shampoo wurde durch Vermischen der folgenden Bestandteile hergestellt.

| Bestandteil | Gew.% |
|---|---|
| Natriumlaurethsulfat | 8,0 |
| Lauramidopropylbetain | 1,0 |
| Cocamid-DEA | 3,0 |
| Glycolstearat | 2,0 |
| Polyquaternium 10 | 0,3 |
| Propylenglycol | 3,0 |
| Natriumbenzoat | 0,5 |
| Wasser | 82,2 |
| Summe | 100,0 |

## Beispiel 2

Gasraumanalyse eines gewaschenen Haarmusters (bei 25 bzw. 50°C)

Es wurden Shampoos hergestellt, indem 0,015 Gew% eines erfindungsgemäßen Riechstoffes zu einer Shampooformulierung gemäß Beispiel 1 zugegeben wurden. 3 g einer homogenen Haarprobe wurden in 200 ml einer 3 Gew%igen Wasserlösung dieses Shampoos (40°C) eingetaucht. Dann wurde dieses Haarmuster gut mit 2 1 Wasser (40°C) gespült und mit einem trockenen Handtuch gut trocken getupft. Dieses Haarmuster wurde in eine 100-ml-Ampulle gelegt, und diese wurde sofort versiegelt. Nach einer 30-minütigen Aufbewahrung dieser Ampulle bei einer fest eingestellten Temperatur (25°C bzw. 50°C) wurde der Gasraum des gewaschenen Haarmusters in dieser Ampulle durch eine 30-minütige Einführung einer Festphasen-Mikroextraktionsfaser (PDMS/DVB 65 $\mu$m, Sigma Aldrich) extrahiert (Gasraum-Probe) und per GC/MS analysiert. Im Gesamtionenchromatogramm wurde eine Peakfläche dieses Riechstoffes aufgezeichnet. Diese Analyse wurde für alle der in Tabelle 2 aufgeführten Riechstoffe durchgeführt.

Bedingungen der Analyse

GC/MS: Agilent Technology, Serie HP6890
Säule: J&W, DB-WAX, 60 m, 0,25 mm Innendurchmesser, 0,25 $\mu$m df
Ofentemperatur: 60 bis 235°C mit einer Heizrate von 4°C/min
Einspritzöffnung: 250°C, Split-Vent für 1 min geschlossen
Detektor Agilent Technology HP5973 MSD

Massenbereich: 27 - 300 amu

Transferleitung: 240°C

Trägergas: Helium (30 cm/s) (Konstantstrom-Modus)

Freisetzungsindex

Der Quotient der so erhaltenen GC-Peakflächen der Gasraum-Proben bei 50°C und bei 25°C wurde ermittelt und auf diese Weise der Freisetzungsindex bestimmt.

**Beispiel 3**

Nach der in Beispiel 2 beschriebenen Methode wurde für Geranylacetat, Citral, Dihydromyrcenol, Benzylacetat, 4-Methylen-3,5,6,6-tetramethyl-2-heptanon, Linalool jeweils der Freisetzungsindex ermittelt (Tabelle 2).

**Beispiel 4**

Bestimmung der Geruchsstärke

Es wurden Shampoos hergestellt, indem 0,015 Gew.% eines erfindungsgemäßen Riechstoffes zu einer Shampooformulierung gemäß Beispiel 1 zugegeben wurden. 3 g einer homogenen Haarprobe wurden in 200 ml einer 3 %igen Wasserlösung dieses Shampoos (40°C) eingetaucht. Dann wurde dieses Haarmuster gut mit 2 l Wasser (40°C) gespült und mit einem trockenen Handtuch gut trocken getupft.

Dieses Haarmuster wurde in ein 1-1-Becherglas gelegt und 1 min lang mittels eines Haartrockners (Matsushita Electric, EH589, 1200 W) bei etwa 50°C getrocknet. Unmittelbar danach wurde das Becherglas versiegelt. Kurz darauf wurde das Becherglas geöffnet, und die Geruchsstärke des vom gewaschenen Haarmuster freigesetzten Riechstoffes bzw. der freigesetzten Riechstoffmischung von einer Sachverständigengruppe von 5 Personen sensorisch bewertet.

Der Mittelwert der ermittelten Ergebnisse wurde berechnet. Diese Auswertung wurde für alle Riechstoffe der Tabelle 2 durchgeführt.

**Beispiel 5**

Bestimmung der Geruchsstärke

Es wurde analog zu Beispiel 4 die Geruchsstärke für Dimethylbenzylcarbinylbutyrat, Citral, Dihydromyrcenol, Benzylacetat, 4-Methylen-3,5,6,6-tetramethyl-2-heptan und Linalool ermittelt (Tabelle 2).

Die Ergebnisse der Beispiele 2 bis 5 sind in Tabelle 2 zusammengefasst.

**Tabelle 2**

| | Riechstoff | Freisetzungsindex | Geruchsstärke |
|---|---|---|---|
| Beispiel 2 und Beispiel4 | 5-Hexyldihydro-5-methyl-2(3H)-furanon | 11,5 | 4,0 |
| | Acetylcedren | I1,2 | .3,2 |
| | Dodecahydro-3a,6,6,9a-tetramethyl Naphtho [2,1-b] furan | 11,1 | 4,4 |
| | 1-(2-Benzofuranyl)ethanon | 10,3 | 3,4 |
| | 2-Propenylcyclohexyloxyacetat | 10,3 | 3,4 |
| | Oxacyclohexadecen-2-on | 10,2 | 4,0 |
| | a-Hexylzimtaldehyd | 10,1 | 3,0 |
| | Cyclopentadecanolid | 10,0 | 4,0 |
| | 1-(1,2,3,4,5,6,7,8-Octahydro-2,3,8,8-tetramethyl-2-naphthyl)ethanon und 1-(1,2,3,4,5,6,7,8-Octahydro-2,3,5,5-tetramethyl-2-naphthyl)ethanon | 10,0 | 3,4 |
| | 3-Methylcyclopentadecenon | 9,6 | 4,6 |
| | Methyl-2-pentyl-3-oxocyclopentylacetat | 9,3 | 3,0 |
| | Alpha-Iron | 9,2 | 4,0 |
| | 2-Methyl-3-(4-tert.-butylphenyl)propanal | 9,1 | 4,0 |
| | 3-(4-tert.-Butylphenyl)propanal | 7,7 | 3,2 |
| | 3-(3-Isopropylphenyl)butanal | 7,6 | 4,0 |
| | Gamma-Undecalacton | 7,6 | 4,0 |
| | 4-(4-Methyl-3-penten-1-yl)-3-cyclohexen-1-carboxaldehyd | 7,3 | 3,4 |
| | 1-(3-Methyl-2-benzofuranyl)ethanon | 7,1 | 4,4 |
| | 2,6,10-Trimethyl-9-undecenal | 6,9 | 4,0 |
| | Beta-Ionon | 6,8 | 4,2 |
| | Heliotropin | 6,5 | 4,0 |
| | Gamma-Methylionon | 6,2 | 4,4 |
| | 2-Methyl-3-(4-isopropyl Phenyl)propanal | 5,9 | 3,0 |
| | Acetaldehyd-2-phenyl-2,4-pentandiolacetal | 5,7 | 3,2 |
| | Dodecanal | 5,0 | 3,8 |
| | Beta-Damascon | 4,8 | 3,4 |
| | Beta-Damascenon | 4,3 | 3,8 |
| | Delta-Damascon | 4,1 | 4,0 |
| Beispiel 3 und Beispiel 5 | Dimethylbenzylcarbinylbutyrat | 3,8 | 1,8 |
| | Citral | 3,3 | 2,2 |
| | Benzylacetat | 2,8 | 2,0 |
| | Linalool | 1,3 | 2,2 |
| | 4-Methylen-3,5,6,6-tetramethyl-2-heptan | 1,1 | 1,4 |
| | Dihydromyrcenol | 1,1 | 0,6 |

In Tabelle 2 zeigt der Freisetzungsindex der erfindungsgemäß bevorzugten Riechstoffe (Beispiele 2 und 4) deutlich, dass die Flüchtigkeiten dieser Riechstoffe aus einem gewaschenen Haarmuster durch Erwärmen von 25°C auf 50°C überraschenderweise bemerkenswert zunahmen. Dieser Effekt wurde für Werte des Freisetzungsindex von 4 und höher gefunden. Weiterhin wurde gefunden, dass die Geruchsstärke der erfindungsgemäß bevorzugten Riechstoffe beim Trocknen des Haares mit einem Haartrockner bei etwa 50°C einen überraschend hohen Wert hat. Hierbei wurden Geruchsstärken von 2,5 und höher gefunden.

Hingegen zeigen die Riechstoffe aus Beispiel 3 und Beispiel 5 sowohl einen niedrigen Freisetzungsindex von höchstens 3,8 sowie eine geringe Geruchsstärke von höchstens 2,2.

## Beispiel 6 und Beispiel 7

Es wurden zwei Riechstoffmischungen zusammengemischt, die die in Tabelle 3 aufgeführten Zusammensetzungen hatten. Diese Riechstoffmischungen weisen insgesamt einen blumigen Duft auf, sowohl in Substanz als auch auf dem Haar während und nach dem Trocknen.

**Tabelle 3**

|   | Riechstoff Gew.% | Beispiel 6 | Beispiel 7 |
|---|---|---|---|
|   | o-Tert.-Butylcyclohexylacetat | 5,00 | 5,00 |
|   | Benzylacetat | 4,00 | 12,00 |
|   | Benzylalkohol |  | 20,00 |
|   | Benzylsalicylat | 4,00 | 4,00 |
|   | Citronellol | 2,00 | 2,00 |
|   | Citronellylacetat | 1,00 | 1,00 |
|   | Alpha-Damascon |  | 0,10 |
|   | Dipropylenglycol | 0,90 | 0,90 |
|   | Dihydromyrcenol | 4,00 | 4,00 |
|   | Ethylenbrassylat | 5,00 | 5,00 |
|   | Geranylacetat | 0,70 | 0,70 |
|   | Cis-3-Hexenol | 0,10 | 0,10 |
|   | Cis-3-Hexenylacetat | 0,10 | 0,10 |
|   | Limonen | 18,00 | 18,00 |
|   | Linalool | 7,00 | 7,00 |
|   | Linalylacetat | 6,00 | 6,00 |
|   | 4(3)-(4-Hydroxy-4-methylpentyl)-3-cyclohexen-1-carboxaldehyd | 6,00 | 6,00 |
|   | Phenylethylalkohol | 8,00 | 8,00 |
|   | Dimethyltetrahydrobenzaldehyd | 0,10 | 0,10 |
|   | Isopropylmyristat | 0,90 |  |
| * | Methyl-2-pentyl-3-oxocyclopentylacetat | 8,00 |  |
| * | Dodecahydro-3a,6,6,9a-tetramethylnaphtho[2,1-b]furan | 0,10 |  |
| * | Cyclopentadecanolid | 3,00 |  |
| * | Beta-Damascenon | 0,10 |  |
| * | Gamma-Methylionon | 5,00 |  |
| * | 2-Methyl-3-(4-tert.-butylphenyl)propanal | 8,00 |  |
| * | Acetylcedren | 3,00 |  |
|   | Summe | 100,0 | 100,0 |

Die Riechstoffmischung aus Beispiel 7 ist eine traditionelle Riechstoffmischung welche so oder in ähnlicher Art in zahlreichen Shampoos zum Einsatz kommt. Hingegen wurde die Riechstoffmischung aus Beispiel 6 mit den erfindungsgemäß bevorzugten Riechstoffen (*) erweitert und dafür andere Riechstoffe in den Gewichtsprozenten reduziert. Hierbei wurde der Geruchstyp der Riechstoffmischung nicht wesentlich verändert.

**Beispiel 8: Haarspülung**

Eine Haarspülung enthaltend die Riechstoffmischung gemäß Beispiel 6 wurde durch das Mischen folgender Bestandteile hergestellt.

| Bestandteil | Gew.% |
|---|---|
| Mineralöl | 2,0 |
| Cetylalkohol | 1,5 |
| Stearylalkohol | 0,8 |
| Steartrimoniumchlorid | 0,8 |
| Glycerin | 3,0 |
| Methylparaben | 0,1 |
| Riechstoffmischung Beispiel 6 | 0,5 |
| Wasser | 91,3 |
| Summe | 100,0 |

**Beispiel 9**

Eine Haarspülung enthaltend die Riechstoffmischung aus Beispiel 7 wurde analog zu Beispiel 8 hergestellt.

**Beispiel 10**

20 g eines homogenen Haarbündels wurden in Wasser (40°C) eingetaucht, und 1 g der in Beispiel 8 hergestellten Spülung wurde auf dieses Haarbündel aufgebracht. Dann wurde dieses Haarbündel gut mit Wasser (40°C) gespült und mit einem trockenen Handtuch gut trocken getupft. Dieses Haarbündel wurde in ein 10-1-Becherglas gelegt, und dieses wurde sofort verschlossen. Danach wurde das Becherglas geöffnet, und die Geruchsstärke der von dem gespülten Haarbündel freigesetzten Riechstoffmischung wurde von einer Expertengruppe von 5 Personen auf dieselbe Weise wie in Beispiel 4 beurteilt. (Auswertung vor dem Trocknen).

Danach wurde das Haarbündel 1 min lang mittels eines Haartrockners (Matsushita Electric, EH589, 1200 W) getrocknet, und dann wurde das Becherglas wiederum sofort versiegelt. Danach wurde das Becherglas geöffnet, und die Geruchsstärke der von dem gespülten Haarbündel freigesetzten Riechstoffmischung wurde von einer Expertengruppen von 5 Personen auf dieselbe Weise wie in Beispiel 4 beurteilt. (Auswertung nach dem Trocknen). Die Mittelwerte der Ergebnisse wurden berechnet.

**Beispiel 11**

Analog zu Beispiel 10 wurde mit der Haarspülung aus Beispiel 9 verfahren.

Die Ergebnisse der Beispiele 10 und 11 sind in Tabelle 4 zusammengefasst.

**Tabelle 4**

| | Geruchsstärke vor dem Trocknen | Geruchsstärke nach dem Trocknen |
|---|---|---|
| Beispiel 10 | 1,0 | 4,2 |
| Beispiel 11 | 1,2 | 1,8 |

Tabelle 4 zeigt, dass die erfindungsgemäße Riechstoffmischung aus Beispiel 10, welche erfindungsgemäß bevorzugte Riechstoffe enthält, vor dem Trocknen eine vergleichbare Geruchsstärke wie die Riechstoffmischung des Beispiels 11 hat. Dagegen wird beim Trocknen des Haares mittels eines Haartrockners und nach dem Trocknen eine deutlich gesteigerte Geruchsstärke durch die Sachverständigengruppe für das erfindungsgemäße Beispiel 10 gefunden. Die Riechstoffmischung aus Beispiel 10 wurde von einer Anwendergruppe deutlich bevorzugt.

**Patentansprüche**

1.  Haarpflegemittel enthaltend eine Riechstoffmischung enthaltend mindestens 5 Riechstoffe ausgewählt aus

    - den Riechstoffen der Gruppe A: Acetaldehyd-2-phenyl-2,4-pentandiolacetal, 2,6,10-Trimethyl-9-undecenal, 2-Propenylcyclohexyloxyacetat, Dodecanal, 1-(3-Methyl-2-benzofuranyl)ethanon, und/oder
    - den Riechstoffen der Gruppe B: Gamma-Undecalacton, 3-(4-tert-Butylphenyl)propanal, 4-(4-Methyl-3-penten-1-yl)-3-cyclohexen-1-carboxaldehyd, 2-Methyl-3-(4-tert.-butylphenyl)propanal, 2-Methyl-3-(4-isopropylphenyl) propanal, 3-(3-Isopropylphenyl)butanal, Beta-Damascenon, Beta-Damascon, Delta-Damascon, 5-Hexyldihydro-5-methyl-2(3H)-furanon, Alpha-Hexylzimtaldehyd, Alpha-Iron, Beta-Ionon, Gamma-Methylionon, Methyl-2-pentyl-3-oxocyclopentylacetat, 1-(2-Benzofuranyl)ethanon, Heliotropin, und/oder
    - den Riechstoffen der Gruppe C: 3-Methylcyclopentadecenon, 1-(1,2,3,4,5,6,7,8-Octahydro-2,3,8,8-tetramethyl-2-naphthyl)ethanon, 1-(1,2,3,4,5,6,7,8-Octahydro-2,3,5,5-tetramethyl-2-naphthyl)ethanon, Acetylcedren, Oxacyclohexadecen-2-on, Dodecahydro-3a,6,6,9a-tetramethylnaphtho[2,1-b]furan, Cyclopentadecanolid,

    mit der Maßgabe, dass der Gesamtanteil dieser Riechstoffe an der Gesamt-Riechstoffmischung 30 bis 100 Gew.-% beträgt.

2.  Haarpflegemittel nach Anspruch 1, **dadurch gekennzeichnet, dass** dieses mindestens 6 Riechstoffe ausgewählt aus den Riechstoffen der Gruppen A, B und/oder C enthält.

3.  Haarpflegemittel nach einem der vorherigen Ansprüche, **dadurch gekennzeichnet, dass** die Summe der Riechstoffe aus den Gruppen A, B und/oder C mindestens 40 Gew.% der Gesamt-Riechstoffmischung beträgt.

4.  Haarpflegemittel nach einem der vorherigen Ansprüche, **dadurch gekennzeichnet, dass** die Riechstoffmischung zumindest zwei Riechstoffe der Gruppe B und zumindest einen Riechstoff der Gruppe A und/oder zumindest einen Riechstoff der Gruppe C aufweist.

5.  Haarpflegemittel nach einem der vorherigen Ansprüche, **dadurch gekennzeichnet, dass**

    a) wenn die Riechstoffmischung Riechstoffe der Gruppen A, B und C enthält: das Gewichtsverhältnis von A : B von 1 : 2 bis 1 : 4 beträgt und der Verhältnis von B : C 1 :1 bis 1: 4 beträgt,
    b) wenn die Riechstoffmischung nur Riechstoffe der Gruppen A und B enthält: das Gewichtsverhältnis von A : B von 1 : 1 bis 1 : 4 beträgt, und
    c) wenn die Riechstoffmischung nur Riechstoffe der Gruppen B und C enthält: das Gewichtsverhältnis von B : C von 1 : 2 bis 5 : 1 beträgt.

6.  Haarpflegemittel nach einem der vorherigen Ansprüche, **dadurch gekennzeichnet, dass** die Riechstoffmischung zumindest einen Riechstoff enthält ausgewählt aus der Gruppe A: 2,6,10-Trimethyl-9-undecenal, 2-Propenylcyclohexyloxyacetat und 1-(3-Methyl-2-benzofuranyl)ethanon;
    mindestens zwei Riechstoffe enthält der Gruppe B: Gamma-Undecalacton, 2-Methyl-3-(4-tert.-butylphenyl)propanal, 3-(3-Isopropylphenyl)butanal, 5-Hexyldihydro-5-methyl-2(3H)-furanon, Alpha-Hexylzimtaldehyd, Alpha-Iron, Beta-Ionon, Gamma-Methylionon, Methyl-2-pentyl-3-oxocyclopentylacetat, 1-(2-Benzofuranyl)ethanon und Heliotropin;
    und mindestens einen Riechstoff enthält der Gruppe C: 3-Methylcyclopentadecenon, 1-(1,2,3,4,5.6,7,8-Octahydro-2,3,8,8-tetramelhyl-2-naphthyl)ethanon, 1-(1,2,3,4,5,6,7,8-Octahydro-2,3,5,5-tetramethyl-2-naphthyl)-ethanon, Acetylcedren, Oxacyclohexadecen-2-on, Dodecahydro-3a,6,6,9a-tetramethylnaphtho[2,1-b]furan und Cyclopentadecanolid.

7.  Verwendung einer Riechstoffmischung in einem Haarpflegemittel, wobei die Riechstoffmischung mindestens 5 Riechstoffe enthält ausgewählt aus

    - den Riechstoffen der Gruppe A: Acetaldehyd-2-phenyl-2,4-pentandiolacetal, 2,6,10-Trimethyl-9-undecenal, 2-Propenylcyclohexyloxyacetat, Dodecanal, 1-(3-Methyl-2-benzofuranyl)ethanon, und/oder
- den Riechstoffen der Gruppe B: Gamma-Undecalacton, 3-(4-tert.-Butylphenyl)propanal, 4-(4-Methyl-3-penten-1-yl)-3-cyclohexen-1-carboxaldehyd, 2-Methyl-3-(4-tert.-butylphenyl)propanal, 2-Methyl-3-(4-isopropylphenyl)propanal, 3-(3-Isopropylphenyl)butanal, Beta-Damascenon, Beta-Damascon, Delta-Damascon, 5-Hexyldihydro-5-methyl-2(3H)-furanon, Alpha-Hexylzimtaldehyd, Alpha-Iron, Beta-Ionon, Gamma-Methylionon, Methyl-2-pentyl-3-oxocyclopentylacetat, 1-(2-Benzofuranyl)ethanon, Heliotropin, und/oder
- den Riechstoffen der Gruppe C: 3-Methylcyclopentadecenon, 1-(1,2,3,4,5,6,7,8-Octahydro-2,3,8,8-tetramethyl-2-naphthyl)ethanon, 1-(1,2,3,4,5,6,7,8-Octahydro-2,3,5,5-tetramethyl-2-naphthyl)ethanon, Acetylcedren, Oxacyclohexadecen-2-on, Dodecahydro-3a,6,6,9a-tetramethylnaphtho[2,1-b]furan, Cyclopentadecanolid,

mit der Maßgabe, dass der Gesamtanteil dieser Riechstoffe an der Gesamt-Riechstoffmischung 30 bis 100 Gew.-% beträgt.

**8.** Verwendung nach einem der vorherigen Ansprüche, wobei die Riechstoffmischung mindestens 6 Riechstoffe enthält ausgewählt aus den Riechstoffen der Gruppen A, B und/oder C.

**9.** Verwendung nach einem der Ansprüche 7 oder 8, **dadurch gekennzeichnet, dass** die Summe der Riechstoffe aus den Gruppen A, B, und/oder C mindestens 40 Gew.% der Gesämt-Riechstoffmischung beträgt.

**10.** Verwendung nach einem der Ansprüche 7 oder 8, **dadurch gekennzeichnet, dass** die Riechstoffmischung zumindest zwei Riechstoffe der Gruppe B und zumindest einen Riechstoff der Gruppe A und/oder zumindest einen Riechstoff der Gruppe C aufweist.

**11.** Verwendung nach einem der Ansprüche 7 oder 8, **dadurch gekennzeichnet, dass**

a) wenn die Riechstoffmischung Riechstoffe der Gruppen A, B und C enthält: das Gewichtsverhältnis von A : B von 1 : 2 bis 1 : 4 beträgt und der Verhältnis von B : C 1 : 1 bis 1 : 4 beträgt,
b) wenn die Riechstoffmischung nur Riechstoffe der Gruppen A und B enthält: das Gewichtsverhältnis von A : B von 1 : 1 bis 1 : 4 beträgt, und
c) wenn die Riechstoffmischung nur Riechstoffe der Gruppen B und C enthält: das Gewichtsverhältnis von B : C von 1 : 2 bis 5 : 1 beträgt.

**12.** Verwendung nach einem der Ansprüche 7 oder 8, **dadurch gekennzeichnet, dass** die Riechstoffmischung zumindest einen Riechstoff enthält ausgewählt aus der Gruppe A: 2,6,10-Trimethyl-9-undecenal, 2-Propenylcyclohexyloxyacetat und 1-(3-Methyl-2-benzofuranyl)ethanon;
mindestens zwei Riechstoffe enthält der Gruppe B: Gamma-Undecalacton, 2-Methyl-3-(4-tert.-butylphenyl)propanal, 3-(3-Isopropylphenyl)butanal, 5-Hexyldihydro-5-methyl-2(3H)-furanon. Alpha-Hexylzimtaldehyd, Alpha-Iron, Beta-Ionon, Gamma-Methylionon, Methyl-2-pentyl-3-oxocyclopentylacetat, 1-(2-Benzofuranyl)ethanon und Heliotropin;
und mindestens einen Riechstoff enthält der Gruppe C: 3-Methylcyclopentadecenon, 1-(1,2,3,4,5,6,7,8-Octahydro-2,3,8,8-tetramethyl-2-naphthyl)ethanon, 1-(1,2,3,4,5.6,7,8-Octahydro-2,3,5,5-tetramethyl-2-naphthy1)-ethanon, Acetylcedren, Oxacyclohexadecen-2-on, Dodecahydro-3a,6,6,9a-tetramethylnaphtho[2,1-b]furan und Cyclopentadecanolid.

## Claims

**1.** Hair care product comprising a fragrance mixture containing at least 5 fragrances selected from

- the following fragrances of group A: acetaldehyde-2-phenyl-2,4-pentanediol acetal, 2,6,10-trimethyl-9-undecenal, 2-propenyl cyclohexyloxyacetate, dodecanal, 1-(3-methyl-2-benzofuranyl)ethanone, and/or
- the following fragrances of group B: gamma-undecalactone, 3-(4-tert-butylphenyl)propanal, 4-(4-methyl-3-penten-1-yl)-3-cyclohexene-1-carboxaldehyde, 2-methyl-3-(4-tert-butylphenyl)propanal, 2-methyl-3-(4-isopropylphenyl)propanal, 3-(3-isopropylphenyl)butanal, beta-damascenone, beta-damascone, delta-damascone, 5-hexyl-dihydro-5-methyl-2(3H)-furanone, alpha-hexylcinnamaldehyde, alpha-irone, beta-ionone, gamma-methylionone, methyl 2-pentyl-3-oxocyclopentylacetate, 1-(2-benzofuranyl)ethanone, heliotropin, and/or
- the following fragrances of group C: 3-methylcyclo-pentadecenone, 1-(1,2,3,4,5,6,7,8-octahydro-2,3,8,8-tetramethyl-2-naphthyl)ethanone, 1-(1,2,3,4,5,6,7,8-octahydro-2,3,5,5-tetramethyl-2-naphthyl)ethanone, acetylcedrene, oxacyclohexadecen-2-one, dodecahydro-3a,6,6,9a-tetramethylnaphtho[2,1-b]furan, cyclopentadecanolide, with the proviso that the total proportion of these fragrances in the overall fragrance mixture is 30 to 100 wt.%.

2. Hair care product according to Claim 1, **characterized in that** it contains at least 6 fragrances selected from the fragrances of groups A, B and/or C.

3. Hair care product according to one of the preceding claims, **characterized in that** the sum of the fragrances from groups A, B and/or C is at least 40 wt.% of the overall fragrance mixture.

4. Hair care product according to one of the preceding claims, **characterized in that** the fragrance mixture contains at least two fragrances from group B and at least one fragrance from group A and/or at least one fragrance from group C.

5. Hair care product according to one of the preceding claims, **characterized in that**

> a) if the fragrance mixture contains fragrances from groups A, B and C, the weight ratio A:B is 1:2 to 1:4 and the ratio B:C is 1:1 to 1:4,
> b) if the fragrance mixture contains fragrances only from groups A and B, the weight ratio A:B is 1:1 to 1:4, and
> c) if the fragrance mixture contains fragrances only from groups B and C, the weight ratio B:C is 1:2 to 5:1.

6. Hair care product according to one of the preceding claims, **characterized in that** the fragrance mixture contains at least one fragrance selected from group A: 2,6,10-trimethyl-9-undecenal, 2-propenyl cyclohexyloxyacetate and 1-(3-methyl-2-benzofuranyl)ethanone;

at least two fragrances from group B: gamma-undecalactone, 2-methyl-3-(4-tert-butylphenyl)propanal, 3-(3-isopropylphenyl)butanal, 5-hexyldihydro-5-methyl-2(3H)-furanone, alpha-hexylcinnamaldehyde, alpha-irone, beta-ionone, gamma-methylionone, methyl 2-pentyl-3-oxocyclopentylacetate, 1-(2-benzofuranyl)ethanone and heliotropin;

and at least one fragrance from group C: 3-methylcyclopentadecenone, 1-(1,2,3,4,5,6,7,8-octahydro-2,3,8,8-tetramethyl-2-naphthyl)ethanone, 1-(1,2,3,4,5,6,7,8-octahydro-2,3,5,5-tetramethyl-2-naphthyl)ethanone, acetylcedrene, oxacyclohexadecen-2-one, dodecahydro-3a,6,6,9a-tetramethylnaphtho[2,1-b]furan and cyclopentadecanolide.

7. Use of a fragrance mixture in a hair care product, the fragrance mixture containing at least 5 fragrances selected from

> - the following fragrances of group A: acetaldehyde-2-phenyl-2,4-pentanediol acetal, 2,6,10-trimethyl-9-undecenal, 2-propenyl cyclohexyloxyacetate, dodecanal, 1-(3-methyl-2-benzofuranyl)ethanone, and/or
> - the following fragrances of group B: gamma-undecalactone, 3-(4-tert-butylphenyl)propanal, 4-(4-methyl-3-penten-1-yl)-3-cyclohexene-1-carboxaldehyde, 2-methyl-3-(4-tert-butylphenyl)propanal, 2-methyl-3-(4-isopropylphenyl)propanal, 3-(3-isopropylphenyl)butanal, beta-damascenone, beta-damascone, delta-damascone, 5-hexyl-dihydro-5-methyl-2(3H)-furanone, alpha-hexylcinnamaldehyde, alpha-irone, beta-ionone, gamma-methylionone, methyl 2-pentyl-3-oxocyclopentylacetate, 1-(2-benzofuranyl)ethanone, heliotropin, and/or
> - the following fragrances of group C: 3-methylcyclopentadecenone, 1-(1,2,3,4,5,6,7,8-octahydro-2,3,8,8-tetramethyl-2-naphthyl)ethanone, 1-(1,2,3,4,5,6,7,8-octahydro-2,3,5,5-tetramethyl-2-naphthyl)ethanone, acetylcedrene, oxacyclohexadecen-2-one, dodecahydro-3a,6,6,9a-tetramethylnaphtho[2,1-b]furan, cyclopentadecanolide, with the proviso that the total proportion of these fragrances in the overall fragrance mixture is 30 to 100 wt.%.

8. Use according to one of the preceding claims, the fragrance mixture containing at least 6 fragrances selected from the fragrances of groups A, B and/or C.

9. Use according to Claim 7 or 8, **characterized in that** the sum of the fragrances from groups A, B and/or C is at least 40 wt.% of the overall fragrance mixture.

10. Use according to Claim 7 or 8, **characterized in that** the fragrance mixture contains at least two fragrances from group B and at least one fragrance from group A and/or at least one fragrance from group C.

11. Use according to Claim 7 or 8, **characterized in that**

a) if the fragrance mixture contains fragrances from groups A, B and C, the weight ratio A:B is 1:2 to 1:4 and the ratio B:C is 1:1 to 1:4,

b) if the fragrance mixture contains fragrances only from groups A and B, the weight ratio A:B is 1:1 to 1:4, and

c) if the fragrance mixture contains fragrances only from groups B and C, the weight ratio B:C is 1:2 to 5:1.

**12.** Use according to Claim 7 or 8, **characterized in that** the fragrance mixture contains

at least one fragrance selected from group A: 2,6,10-trimethyl-9-undecenal, 2-propenyl cyclohexyloxyacetate and 1-(3-methyl-2-benzofuranyl)ethanone;

at least two fragrances from group B: gamma-undecalactone, 2-methyl-3-(4-tert-butylphenyl)propanal, 3-(3-isopropylphenyl)butanal, 5-hexyldihydro-5-methyl-2(3H)-furanone, alpha-hexylcinnamaldehyde, alpha-irone, beta-ionone, gamma-methylionone, methyl 2-pentyl-3-oxocyclopentylacetate, 1-(2-benzofuranyl)ethanone and heliotropin;

and at least one fragrance from group C: 3-methylcyclopentadecenone, 1-(1,2,3,4,5,6,7,8-octahydro-2,3,8,8-tetramethyl-2-naphthyl)ethanone, 1-(1,2,3,4,5,6,7,8-octahydro-2,3,5,5-tetramethyl-2-naphthyl)ethanone, acetylcedrene, oxacyclohexadecen-2-one, dodecahydro-3a,6,6,9a-tetramethylnaphtho[2,1-b]furan and cyclopentadecanolide.

## Revendications

**1.** Produit pour soins de la chevelure contenant un mélange de matières odorantes contenant lui-même au moins 5 matières odorantes choisies parmi

- les matières odorantes du groupe A : acétaldéhyde-2-phényl-2,4-pentanediolacétal, 2,6,10-triméthyl-9-undécénal, 2-propénylcyclohexyloxyacétate, dodécanal, 1-(3-méthyl-2-benzofurannyl)éthanone, et/ou
- les matières odorantes du groupe B : gamma-undécalactone, 3-(4-tert.-butylphényl)propanal, 4-(4-méthyl-3-pentén-1-yl)-3-cyclohexène-1-carboxaldéhyde, 2-méthyl-3-(4-tert.-butylphényl)propanal, 2-méthyl-3-(4-isopropylphényl)propanal, 3-(3-isopropylphényl)butanal, bêta-damascénone, bêta-damascone, delta-damascone, 5-hexyldihydro-5-méthyl-2(3H)-furannone, aldéhyde alpha-hexylcinnamique, alpha-irone, bêta-ionone, gamma-méthylionone, méthyl-2-pentyl-3-oxocyclopentylacétate, 1-(2-benzofurannyl)éthanone, héliotropine, et/ou
- les matières odorantes du groupe C : 3-méthylcyclopentadécénone, 1-(1,2,3,4,5,6,7,8-octahydro-2,3,8,8-tétraméthyl-2-naphtyl)-éthanone, 1-(1,2,3,4,5,6,7,8-octahydro-2,3,5,5-tétraméthyl-2-naphtyl)-éthanone, acétylcédrène, oxacyclohexadécène-2-one, dodécahydro-3a,6,6,9a-tétraméthylnaphto[2,1-b]furanne, cyclopentadécanolide, sous réserve que la proportion totale de ces matières odorantes représente de 30 à 100 % du poids du mélange de matières odorantes.

**2.** Produit pour soins de la chevelure selon la revendication 1, **caractérisé en ce qu'**il contient au moins 6 matières odorantes choisies parmi les matières odorantes des groupes A, B et/ou C.

**3.** Produit pour soins de la chevelure selon une des revendications qui précèdent, **caractérisé en ce que** la somme des matières odorantes des groupes A, B et/ou C représente au moins 40 % du poids du mélange total de matières odorantes.

**4.** Produit pour soins de la chevelure selon une des revendications qui précèdent, **caractérisé en ce que** le mélange de matières odorantes contient au moins deux matières odorantes du groupe B et au moins une matière odorante du groupe A et/ou au moins une matière odorante du groupe C.

**5.** Produit pour soins de la chevelure selon une des revendications qui précèdent, **caractérisé en ce que**

a) lorsque le mélange de matières odorantes contient des matières odorantes des groupes A, B et C, les proportions relatives en poids A:B vont de 1:2 à 1:4 et les proportions relatives B:C vont de 1:1 à 1:4,

b) lorsque le mélange de matières odorantes ne contient que des matières odorantes des groupes A et B, les proportions relatives en poids A:B vont de 1:1 à 1:4, et

c) lorsque le mélange de matières odorantes ne contient que des matières odorantes des groupes B et C, les proportions relatives en poids B:C vont de 1:2 à 5:1.

**6.** Produit pour soins de la chevelure selon l'une des revendications qui précèdent, **caractérisé en ce que** :

il contient au moins une matière odorante prise dans le groupe A : 2,6,10-triméthyl-9-undécénal, 2-propénylcyclohexyloxyacétate et 1-(3-méthyl-2-benzofurannyl)éthanone ;
il contient au moins deux matières odorantes prises dans le groupe B : gamma-undécalactone, 2-méthyl-3-(4-tert.-butylphényl)propanal, 3-(3-isopropylphényl)butanal, 5-hexyldihydro-5-méthyl-2(3H)-furannone, aldéhyde alpha-hexylcinnamique, alpha-irone, bêta-ionone, gamma-méthylionone, acétate de méthyl-2-pentyl-3-oxocyclopentyle, 1-(2-benzofurannyle)éthanone et héliotropine ;
et au moins une matière odorante du groupe C : 3-méthylcyclopentadécénone, 1-(1,2,3,4,5,6,7,8-octahydro-2,3,8,8-tétraméthyl-2-naphtyl)-éthanone, 1-(1,2,3,4,5,6,7,8-octahydro-2,3,5,5-tétraméthyl-2-naphtyl)-éthanone, acétylcédrène, oxacyclohexadécène-2-one, dodécahydro-3a,6,6,9a-tétraméthylnaphto[2,1-b]furanne et cyclopentadécanolide.

7. Utilisation d'un mélange de matières odorantes dans un produit pour soins de la chevelure, le mélange de matières odorantes contenant au moins 5 matières odorantes choisies parmi

- les matières odorantes du groupe A : acétaldéhyde-2-phényl-2,4-pentanediolacétal, 2,6,10-triméthyl-9-undécénal, 2-propénylcyclohexyloxyacétate, dodécanal, 1-(3-méthyl-2-benzofurannyl)éthanone, et/ou
- les matières odorantes du groupe B : gamma-undécalactone, 3-(4-tert.-butylphényl)propanal, 4-(4-méthyl-3-pentén-1-yl)-3-cyclohexène-1-carboxaldéhyde, 2-méthyl-3-(4-tert.-butylphényl)propanal, 2-méthyl-3-(4-isopropylphényl)propanal, 3-(3-isopropylphényl)butanal, bêta-damascénone, bêta-damascone, delta-damascone, 5-hexyldihydro-5-méthyl-2(3H)furannone, aldéhyde alpha-hexylcinnamique, alpha-irone, bêta-ionone, gamma-méthylionone, méthyl-2-pentyl-3-oxocyclopentylacétate, 1-(2-benzofurannyl)éthanone, héliotropine, et/ou
- les matières odorantes du groupe C : 3-méthylcyclopentadécénone, 1-(1,2,3,4,5,6,7,8-octahydro-2,3,8,8-tétraméthyl-2-naphtyl)éthanone, 1-(1,2,3,4,5,6,7,8-octahydro-2,3,5,5-tétraméthyl-2-naphtyl)éthanone, acétylcédrène, oxacyclohexadécène-2-one, dodécahydro-3a,6,6,9a-tétraméthylnaphto[2,1-b]furanne, cyclopentadécanolide, sous réserve que la proportion totale de ces matières odorantes représente de 30 à 100 % du poids du mélange total de matières odorantes.

8. Utilisation selon l'une des revendications qui précèdent, le mélange de matières odorantes contenant au moins 6 matières odorantes choisies parmi celles des groupes A, B et/ou C.

9. Utilisation selon l'une des revendications 7 ou 8, **caractérisée en ce que** la somme des matières odorantes des groupes A, B et/ou C représente au moins 40 % du poids du mélange total de matières odorantes.

10. Utilisation selon l'une des revendications 7 ou 8, **caractérisée en ce que** le mélange de matières odorantes contient au moins deux matières odorantes du groupe B et au moins une matière odorante du groupe A et/ou au moins une matière odorante du groupe C.

11. Utilisation selon la revendication 7 ou 8, **caractérisée en ce que**

a) lorsque le mélange de matières odorantes contient des matières odorantes des groupes A, B et C, les proportions relatives en poids A:B vont de 1:2 à 1:4 et les proportions relatives B:C vont de 1:1 à 1:4,
b) lorsque le mélange de matières odorantes ne contient que des matières odorantes des groupes A et B, les proportions relatives en poids A: B vont de 1:1 à 1:4, et
c) lorsque le mélange de matières odorantes ne contient que des matières odorantes des groupes B et C, les proportions relatives en poids B:C vont de 1:2 à 5:1.

12. Utilisation selon l'une des revendications 7 ou 8, **caractérisée en ce que** le mélange de matières odorantes contient au moins une matière odorante choisie parmi celles du groupe A : 2,6,10-triméthyl-9-undécénal, 2-propénylcyclohexyloxyacétate et 1-(3-méthyl-2-benzofurannyl)éthanone ;
au moins deux matières odorantes du groupe B : gamma-undécalactone, 2-méthyl-3-(4-tert.-butylphényl)propanal, 3-(3-isopropylphényl)butanal, 5-hexyldihydro-5-méthyl-2(3H)-furannone, aldéhyde alpha-hexylcinnamique, alpha-irone, bêta-ionone, gamma-méthylionone, méthyl-2-pentyl-3-oxocyclopentylacétate, 1-(2-benzofurannyl)éthanone et héliotropine ;
et au moins une matière odorante du groupe C : 3-méthylcyclopentadécénone, 1-(1,2,3,4,5,6,7,8-octahydro-2,3,8,8-tétraméthyl-2-naphtyl)-éthanone, 1-(1,2,3,4,5,6,7,8-octahydro-2,3,5,5-tétraméthyl-2-naphtyl)-éthanone, acétylcédrène, oxacyclohexadécène-2-one, dodécahydro-3a,6,6,9a-tétraméthylnaphto[2,1-b]furanne et cyclopentadécanolide.

**EP 1 385 470 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **K. BAUER ; D. GARBE ; H. SURBURG.** Common Fragrance and Flavor Materials. Wiley-VCH, 1997